# EUROPÄISCHE PATENTSCHRIFT

(11) **EP 1 419 134 B1**
(45) Veröffentlichungstag und Bekanntmachung des Hinweises auf die Patenterteilung: **16.07.2008**
(21) Anmeldenummer: 02794543.5
(22) Anmeldetag: 02.08.2002
(51) Int. Cl.: C07C 201/04, C07C 203/00

(54) **VERFAHREN ZUR HERSTELLUNG VON ALKYLNITRITEN UND ALKYLDINITRITEN**
METHOD FOR PRODUCING ALKYL NITRITES AND ALKYL DINITRITES
PROCEDE DE PRODUCTION DE NITRITES D'ALKYLE ET DE DINITRITES D'ALKYLE

(30) Priorität: 03.08.2001 DE 10138152
(43) Veröffentlichungstag der Anmeldung: 19.05.2004
(73) Patentinhaber: BASF SE, 67056 Ludwigshafen (DE)
(72) Erfinder: KUDIS, Steffen, 68167 Mannheim (DE); LOCHTMAN, Rene, 68161 Mannheim (DE); EHRESMANN, Manfred, 67063 Ludwigshafen (DE); KEIL, Michael, 67251 Freinsheim (DE); GEBHARDT, Joachim, 67157 Wachenheim (DE); RACK, Michael, 69123 Heidelberg (DE); SCHIMETZEK, Ralf, 67346 Speyer (DE)
(74) Vertreter: Reitstötter - Kinzebach
(86) Internationale Anmeldenummer: PCT/EP2002/008639
(87) Internationale Veröffentlichungsnummer: WO 2003/014059

(56) Entgegenhaltungen:
- WO-A-01/74754
- DE-A- 2 144 420
- DE-B- 1 043 310
- US-A- 2 714 606
- US-A- 4 980 496

## Beschreibung

Die vorliegende Erfindung betrifft ein kontinuierliches Verfahren zur Herstellung von Alkylnitriten und -dinitriten durch Umsetzung von Alkanolen bzw. Dialkanolen mit Nitritionen in Gegenwart einer wässrigen Mineralsäure.

Alkylnitrite und -dinitrite sind wichtige Nitrosierungsreagenzien in der präparativen Chemie. Sie finden beispielsweise zur Herstellung von Diazonium-Verbindungen aus primären Aminen Anwendung. Insbesondere aromatische Diazonium-Verbindungen stellen wichtige Zwischenstufen dar, da die Diazonium-Gruppe in eine Vielzahl von Substituenten wie Pseudohalogen, Halogen (Sandmeyer-Reaktion, Schiemann-Reaktion), Hydroxy, Mercapto oder Wasserstoff umgewandelt werden kann. Diazonium-Verbindungen dienen ferner als Ausgangsstoffe beim Phenanthren-Ringschluss (Pschorr-Reaktion), für die Herstellung von Arylhydrazonen (Japp-Klingemann-Reaktion), von Arylhydrazinen oder von Azoverbindungen.

Die DE 21 444 20 beschreibt ein diskontinuierliches Verfahren zur Herstellung von Glykolen und Glykolderivaten, indem man Glykole oder Glykolderivate mit Natriumnitrit und Eiswasser verrührt und anschließend Salzsäure hinzufügt, wobei man durch Außenkühlung die Temperatur unter 10 °C hält.

Organic Syntheses Coll. Bd. 2, 108 (1953) beschreibt die Herstellung von n-Butylnitrit, indem man ein Gemisch aus Wasser, Isopropanol und Schwefelsäure zu wässrigem Natriumnitrit gibt. Nachteilig an diesem Verfahren ist die Ausbildung eines Dreiphasengemisches mit festem Natriumsulfat in der dritten Phase.

Die US 4,980,496 beschreibt ein diskontinuierliches Verfahren zur Herstellung von Alkylnitriten, wobei man eine wässrige Lösung eines C₁-C₅-Alkanols mit einem Alkalimetallnitrit mischt, die Mischung auf -10 bis etwa 10 °C kühlt und dann unter Einhaltung der Temperatur Halogenwasserstoffsäure hinzufügt. Die Halogenwasserstoffsäure wird in der Regel zur Erreichung eines vollständigen Umsatzes im Überschuss, insbesondere etwa 4 mol-%, bezogen auf den Alkohol eingesetzt.

Das Verfahren der US 4,980,496 ist in verschiedener Hinsicht problematisch. Zum einen verläuft die Reaktion exotherm, so dass die Einhaltung der Reaktionstemperatur im Bereich von -10 bis 10 °C schwierig ist. Zum anderen neigen Alkylnitrite im stark Sauren zur Zersetzung und zur Bildung von Nebenprodukten, so dass der für die Umsetzung erforderliche Säureüberschuss durch Neutralisation abgefangen werden oder das Produkt unmittelbar von der wässrigen Phase abgetrennt und unter geeigneten Trocknungsbedingungen gelagert werden muss.

Eigene Untersuchungen der Anmelderin wiederum haben gezeigt, dass eine Reduzierung der Säuremenge bei diskontinuierlicher Fahrweise zu einer verstärkten Bildung von Nebenprodukten wie Acetalen und Ketalen und zu einer Verringerung der Ausbeute führt.

Der vorliegenden Erfindung liegt daher die Aufgabe zugrunde, ein Verfahren zur Herstellung von Alkylnitriten und -dinitriten zur Verfügung zu stellen, mit dem diese Verbindungen auf einfache Weise und mit erhöhter Reinheit sowie guter Lagerstabilität hergestellt werden können. Dabei sollte der apparative und präparative Aufwand möglichst gering sein, um die Kosten möglichst niedrig zu halten.

Es wurde überraschend gefunden, dass sich Alkylnitrite und -dinitrite in hoher Reinheit und mit guter Lagerstabilität über der wässrigen Phase herstellen lassen, wenn man ein Alkanol oder Dialkanol mit der wässrigen Lösung einer Mineralsäure mischt, wobei man im Mittel nicht mehr als 1,01 Mol Säureäquivalente je mol Hydroxylgruppe des Alkanols oder Dialkanols einsetzt und zu dieser Mischung kontinuierlich eine wässrige Lösung eines anorganischen Nitrits gibt.

Demnach betrifft die vorliegende Erfindung ein Verfahren zur kontinuierlichen Herstellung von Alkylnitriten und -dinitriten durch Umsetzung eines Alkanols oder Dialkanols mit einem anorganischen Nitrit in Gegenwart von wenigstens einer auf Nitrit nicht oxidierend wirkenden Mineralsäure, dadurch gekennzeichnet, dass man
(i) das Alkanol oder Dialkanol mit einer wässrigen Lösung der Mineralsäure mischt, wobei man im Mittel nicht mehr als 1,01 Mol Säureäquivalente je mol Hydroxylgruppe des Alkanols oder Dialkanols einsetzt,
(ii) in einer Reaktionszone zu in (i) erhaltenem wässrigen Gemisch kontinuierlich eine wässrige Lösung des anorganischen Nitrits gibt, und
(iii) gegebenenfalls die organische Phase isoliert.

Beispiele für auf Nitrit nicht oxidierend wirkende wässrige Mineralsäuren umfassen verdünnte Schwefelsäure, Phosphorsäure sowie Halogenwasserstoffsäuren, z. B. Chlorwasserstoff, Bromwasserstoff oder Iodwasserstoff. In einer bevorzugten Ausführungsform setzt man Chlorwasserstoff in Wasser (=-Salzsäure) ein. Die Konzentration von Chlorwasserstoff in Wasser kann innerhalb weiter Grenzen variiert werden. Im Allgemeinen liegt sie im Bereich von 10 bis 40 Gew.-%, vorzugsweise 15 bis 35 Gew.-%. Üblicherweise setzt man die Salzsäure in technischer Qualität ein.

Geeignete Alkanole und Dialkanole sind solche, die sich in Wasser zumindest begrenzt lösen. Hierzu zählen C₂-C₆-Alkanole, wie Ethanol, n-Propanol, Isopropanol, n-Butanol, sec-Butanol, Isobutanol, tert.-Butanol, n-Amylalkohol, Isoamylalkohol (3-Methyl-1-butanol), 2-Methyl-1-butanol, n-Hexanol, 2-Hexanol und 2-Methyl-1-pentanol.

Geeignete Dialkanole sind ausgewählt unter C₂-C₆-Dialkanolen wie 2,2-Dimethyl-1,3-propandiol, 1,4-Butandiol, 1,5-Pentandiol, speziell 2,2-Dimethyl-1,3-propandiol.

Besonders geeignet ist das erfindungsgemäße Verfahren für die Umsetzung von n-Butanol, tert.-Butanol und Isoamylalkohol.

In der Regel setzt man das Alkanol bzw. das Dialkanol unverdünnt ein. Gegebenenfalls kann man ein Lösungsmittel mitverwenden. Hierbei kommt vorzugsweise Wasser in Betracht. Grundsätzlich können auch mit Wasser mischbare Lösungsmittel eingesetzt werden, die gegenüber saurem Nitrit inert sind und die auch nicht die Verwendung des Reaktionsproduktes einschränken, z. B. cyclische Ether wie Dioxan oder Tetrahydrofuran oder Ketone wie Aceton und Methylethylketon.

Üblicherweise wählt man die Konzentration an wässriger Mineralsäure und Alkanol bzw. Dialkanol so, dass man im Mittel nicht mehr als 1,01 Mol, vorzugsweise 1,00 bis 1,005 Mol Mineralsäureäquivalente je mol Hydroxylgruppe des Alkanols bzw. des Dialkanols einsetzt. In einer ganz besonders bevorzugten Ausführungsform der Erfindung setzt man pro mol Hydroxylgruppe des Alkanols bzw. Dialkanols die Mineralsäure in äquimolarer Menge ein.

Das Mischen von Alkanol und wässriger Mineralsäure kann in üblicher Weise erfolgen. Die Mischtemperatur ist für den erfindungsgemäßen Erfolg von untergeordneter Bedeutung. In der Regel wird man die Mischung bei der für die anschließende Reaktion mit dem Nitrit gewünschten Temperatur herstellen, bei einer Temperatur von vorzugsweise oberhalb 5 bis 40 °C, insbesondere im Bereich von 10 bis 30 °C, speziell 15 bis 25 °C. Gegebenenfalls kühlt man die Mineralsäure und das Alkanol auf die gewünschte Temperatur vor. Das Mischen von Alkanol bzw. Dialkanol und Mineralsäure kann in einem Behälter, in einer Mischkammer oder über statische Mischer in einer Rohrleitung erfolgen. Die Herstellung der Mischung kann bei Verwendung von Mischbehältern sowohl kontinuierlich als auch diskontinuierlich erfolgen. Das Mischen kann durch Rühren oder Strömungsmischen erfolgen. In einer Ausführungsform der Erfindung werden das Alkanol bzw. Dialkanol kontinuierlich unter Durchmischen in einen Behälter eingespeist, vorzugsweise in einen Rührkessel, und diesem kontinuierlich entnommen.

In der zweiten Stufe (ii) des erfindungsgemäßen Verfahrens gibt man in einer Reaktionszone zu in (i) erhaltenem wässrigen Gemisch kontinuierlich eine wässrige Lösung des anorganischen Nitrits.

Geeignete Quellen für Nitritionen sind anorganische Nitrite, z. B. Alkalimetall- und Erdalkalimetallnitrite wie Natrium-, Kalium-, Barium- und Calciumnitrit sowie Ammoniumnitrit. Hiervon sind Kaliumnitrit und speziell Natriumnitrit besonders bevorzugt.

Die Menge an anorganischem Nitrit, welches man in der zweiten Stufe (ii) gelöst in Wasser zuführt, entspricht im Wesentlichen der erforderlichen Stöchiometrie, wobei man das Nitrit auch im Überschuss, der in der Regel 20 Mol-% nicht überschreitet, einsetzen kann. Für die Stöchiometrie wird die eingesetzte Menge an Hydroxylgruppe des Alkanols bzw. Dialkanols zugrunde gelegt. Das molare Verhältnis von Hydroxylgruppe des Alkanols bzw. Dialkanols zu anorganischem Nitrit liegt in der Regel im Bereich von 1:1,0 bis 1:1,2, vorzugsweise 1:1,01 bis 1,2 und insbesondere 1:1,05 bis 1:1,12.

In der Regel beträgt die Gesamtmenge an Wasser in der zweiten Stufe (ii) das 1- bis 5-fache, vorzugsweise das 1,5- bis 4-fache und insbesondere das 2,5- bis 3,5-fache der organischen Phase.

Zur Umsetzung in Schritt (ii) führt man die aus (i) erhaltene Mischung und die wässrige Nitritlösung in eine Reaktionszone, die die gewünschte Reaktionstemperatur aufweist. Bei der Reaktionszone handelt es sich in der Regel um einen oder mehrere, nacheinander geschaltete Reaktionskessel (Kesselkaskade) oder um Reaktionsrohre, wobei Kessel und Kesselkaskade bevorzugt sind. Die Reaktionszonen weisen in der Regel übliche, zum Durchmischen von Flüssigkeiten geeignete Vorrichtungen, z. B. statische Mischvorrichtungen wie Einbauten und/oder Rührer und/oder Vorrichtungen zum Umpumpen von Flüssigkeiten, vorzugsweise in Kombination mit statischen Mischern auf. Eine Durchmischung der Alkohol/Säure-Mischung und der Nitritlösung kann auch dadurch bewirkt werden, dass man die flüssigen Reaktanden über Vorrichtunge, die zur kontinuierlichen Mischen von Flüssigkeiten geeignet sind, der zweiten Stufe (ii) zuführt. Bei kleineren Apparateabmessungen kann ein Durchmischen der Reaktanden auch dadurch erreicht werden, dass man die flüssigen Reaktanden unter Verwirbelung der Flüssigkeitsströme in den Reaktor dosiert, beispielsweise über zwei in unmittelbarer räumlicher Nähe zueinander angeordnete Einlässe für die Flüssigkeitsströme. Diese Vorgehensweise hat sich insbesondere bei Labor- und Miniplant-Anlagen mit Reaktorvolumina (Volumina der jeweiligen Reaktionszone) ≤ 1000 ml, vorzugsweise ≤ 200 ml, bewährt.

Ferner weist die Reaktionszone in der Regel übliche Vorrichtungen zum Abführen von Reaktionswärme, z. B. Kühlschlangen, Wandkühler und dergleichen auf.

Die Verweilzeit der Reaktanden in der Reaktionszone beträgt in der Regel 20 Minuten bis 5 Stunden, vorzugsweise 25 min bis 3 Stunden, insbesondere 40 min bis 2 Stunden und ganz besonders bevorzugt 40 min bis 60 min. Die Verweilzeit ist naturgemäß von der Zulaufgeschwindigkeit der Reaktanden abhängig. Üblicherweise wird man bei niedrigen Reaktionstemperaturen eine längere Verweilzeit und bei höheren Reaktionstemperaturen eine kürzere Verweilzeit wählen.

Die Reaktionstemperatur für die Umwandlung des Alkanols bzw. Dialkanols in das entsprechende Alkylnitrit bzw. -dinitrit in der zweiten Stufe (ii) liegt vorzugsweise im Bereich von oberhalb 5 bis 40 °C, insbesondere 10 bis 30 °C und speziell 15 bis 25 °C.

Das im Austrag der Reaktionszone anfallende Reaktionsgemisch weist in der Regel einen pH-Wert oberhalb 3 auf.

In einer bevorzugten Ausführungsform der Erfindung besteht die Reaktionszone in Stufe (ii) aus wenigstens zwei Reaktionskesseln (Kaskade). Das in Stufe (i) erhaltene Gemisch sowie die wässrige anorganische Nitritlösung werden separat kontinuierlich in einen ersten Reaktor gespeist. Der Inhalt des ersten Reaktors wird in üblicher Weise durchmischt, z. B. durch Rühren, intensives Umpumpen oder durch Eindüsen der Reaktanden. Die Mischung gelangt nach einer durch das Verhältnis von Reaktorvolumen und Zulauf- bzw. Entnahmegeschwindigkeit bedingten Verweilzeit über eine Entnahmevorrichtung, z. B. einen Überlauf, in wenigstens einen weiteren Reaktor zur Vervollständigung der Reaktion. Diese Vorgehensweise hat sich insbesondere bei Reaktorvolumina von wenigstens 200 ml bewährt.

Das Wertprodukt kann gegebenenfalls direkt im Anschluss an Stufe (ii) abgetrennt werden. In der Regel liegt das Reaktionsgemisch zweiphasig vor und trennt sich problemlos. Die Abtrennung des Wertproduktes kann nach den üblichen Techniken erfolgen, beispielsweise durch Eintragen des Reaktionsaustrages aus Schritt (ii) in ein Phasentrenngefäß. Üblicherweise erfolgt das Abtrennen bei Raumtemperatur. Die Schritte (i) bis (iii) werden üblicherweise bei Normaldruck durchgeführt. Das erhaltene Alkylnitrit bzw. -dinitrit weist in der Regel eine GC (FID)-Reinheit größer 95 %, sehr häufig größer 97 % und meist größer 98 % auf. Die Ausbeute an Wertprodukt liegt nur geringfügig unter den erhaltenen GC-Reinheiten.

Das erfindungsgemäße Verfahren hat gegenüber den diskontinuierlichen Verfahren des Standes der Technik den Vorteil, dass das Alkylnitrit bzw. -dinitrit unter Einsatz eines geringeren Säureüberschusses oder ohne Säureüberschuss ohne Einbußen in Produktqualität und Ausbeute hergestellt werden kann, so dass die eingangs geschilderten Nachteile des Standes der Technik überwunden werden. Zudem erlaubt die kontinuierliche Herstellung eine effizientere und raschere Herstellung größerer Produktmengen. Eine Abtrennung der Wertprodukte ist anders als in den diskontinuierlichen Verfahren des Standes der Technik nicht erforderlich, oder nur in den Fällen, in denen der Einsatz von wasserfreiem Alkylnitrit bzw. -dinitrit erforderlich ist. Die Lagerstabilität des erhaltenen Alkylnitrits bzw. -dinitrits ist ebenfalls besser.

Außerdem ist das erfindungsgemäße Verfahren wirtschaftlicher, da anders als bei den diskontinuierlichen Verfahren nicht oder nur geringfügig während der Umsetzung gekühlt werden muss. Die Wasserphase muss zudem nicht unmittelbar im Anschluss an die Reaktion von der Produktphase abgetrennt werden. Vielmehr ist es sogar möglich, die Produktphase über der Wasserphase über einen begrenzten Zeitraum von mehreren Tagen bei Raumtemperatur zu lagern, ohne dass nennenswerte Zersetzung eintritt.

Das erfindungsgemäße Verfahren wird durch die nachfolgenden Beispiele näher erläutert.

### Beispiel 1

In einem 5 l Vorlagebehälter aus Glas wurde n-Butanol und in einem zweiten 5 l Vorlagebehälter aus Glas wurde 20 gew.-%ige Salzsäure vorgelegt und jeweils über eine Membranpumpe im molaren Verhältnis 1:1 in einen 0,75 l Rührreaktor aus Glas gepumpt.

219,5 g/h dieses Gemisches aus n-Butanol und Salzsäure wurden synchron mit einer wässrigen 40 Gew.-%igen Natriumnitritlösung in einen ersten 0,75 l Rührreaktor aus Glas gepumpt. Das molare Verhältnis von n-Butanol zu Salzsäure zu Natriumnitrit betrug 1:1:1,1. Die Mischung gelang nach einer durch die Zulaufzeit bedingten Verweilzeit durch einen Überlauf in einen zweiten 0,75 1 Rührreaktor aus Glas. Die Gesamtverweilzeit der Reaktanden betrug etwa 45 bis 50 min. Durch einen Überlauf gelangte das Reaktionsgemisch schließlich in einen Scheidetrichter. Man trennte das n-Butylnitrit ab, das laut GC (FID) 0,77 % Butanol enthielt. Die Reinheit des Wertproduktes betrug laut GC (FID) 98,87 %. Der Anteil an Dibutoxybutan lag unter 0,1 %. Das Produkt war mehrere Tage über der Wasserphase stabil.

### Beispiel 2:

Man wiederholte Beispiel 1, trennte aber das Butylnitrit erst nach 6 Tagen von der wässrigen Phase ab. Die Lagerung über der wässrigen Phase erfolgte bei 5 °C. Der Zersetzungsgrad des n-Butylnitrits lag unter 0,1 %. Ähnliche Ergebnisse erzielt man bei Lagerung bei 20 °C.

### Beispiel 3:

Man wiederholte Beispiel 1, die Zulaufgeschwindigkeit des Gemisches aus HCl/BuOH betrug aber 324 g/h, und das molare Verhältnis von Butanol:HCl lag bei 1:1,1. Die Reinheit des Wertproduktes betrug laut GC (FID) 98,62 %. Das Wertprodukt enthielt 1,01 % Butanol.

### Beispiel 4:

Man wiederholte Beispiel 3, die Zulaufgeschwindigkeit des Gemisches aus HCl/BuOH betrug aber 233 g/h. Ferner verwendete man eine 32 Gew.-%ige Salzsäure. Die Reinheit des Wertproduktes betrug laut GC (FID) 98,72 %. Das Wertprodukt enthielt 0,93 % Butanol.

### Beispiel 5:

Man wiederholte Beispiel 4, die Zulaufgeschwindigkeit des Gemisches aus HCl/BuOH betrug aber 232 g/h. Außerdem führte man Schritt (ii) bei einer Reaktionsinnentemperatur von 10 °C aus. Die Reinheit des Wertproduktes betrug laut GC (FID) 98,72 %. Das Wertprodukt enthielt 0,97 % Butanol.

### Beispiel 6:

Man wiederholte Beispiel 5, führte aber Schritt (ii) bei einer Reaktionsinnentemperatur von 0 °C aus. Die Reinheit des Wertproduktes betrug laut GC (FID) 99,02 %. Das Wertprodukt enthielt 0,93 % Butanol.

### Beispiel 7 (Vergleichsbeispiel):

Man wiederholte Beispiel 1, die Zulaufgeschwindigkeit des Gemisches aus HCl/BuOH betrug aber 203,5 g/h, und das molare Verhältnis von Butanol:HCl lag bei 1:1,05. Außerdem führte man Schritt (ii) bei einer Reaktionsinnentemperatur von -10 °C aus. Die Reinheit des Wertproduktes betrug laut GC (FID) 98,86 %. Das Wertprodukt enthielt 0,88 % Butanol. Trennte man das Produkt nicht direkt von der wässrigen Phase ab, so trat bei 5 °C Zersetzung zu n-Butanol und Bildung von Dibutoxybutan (bis zu 3 %) innerhalb etwa 2 Tagen ein.

### Beispiel 8 (Vergleich, diskontinuierliche Fahrweise):

In einem Kessel wurden 81,5 kg (1100 mol) n-Butanol vorgelegt und auf -5 °C gekühlt. Während 2,25 h gab man unter Rühren 125,3 kg (1115 mol) 32,4 %ige Salzsäure so zu, dass die Temperatur im Kessel stets unter - 5 °C blieb. Anschließend kühlte man den Kesselinhalt auf -15 °C ab und gab unter Rühren innerhalb etwa 6 h 208,2 kg (1210 mol) einer auf 8 bis 12 °C gekühlten 40%igen Natriummitrit-Lösung hinzu, sodass die Temperatur stets unter - 5 °C blieb. Nach beendeter Zugabe rührte man noch 15 min bei -10 bis -5 °C nach, schaltete zur Phasentrennung den Rührer ab und trennte die organische Phase ab. Die Ausbeute an n-Butylnitrit betrug 98,5 % in einer GC (FID) Reinheit von 98,25 %. Das Produkt enthielt noch 0,73 % Butanol laut GC (FID). Trennte man das Produkt nicht direkt von der wässrigen Phase ab, so trat bei 5 °C Zersetzung zu n-Butanol und Bildung von Dibutoxybutan (bis zu 3 %) innerhalb etwa 2 Tagen ein.

## Patentansprüche

1. Verfahren zur kontinuierlichen Herstellung von Alkylnitriten und Alkyldinitriten durch Umsetzung eines Alkanols oder Dialkanols mit einem anorganischen Nitrit in Gegenwart von wenigstens einer auf Nitrit nicht oxidierend wirkenden Mineralsäure, **dadurch gekennzeichnet, dass** man
(i) das Alkanol oder Dialkanol mit einer wässrigen Lösung der Mineralsäure mischt, wobei man im Mittel nicht mehr als 1,01 Mol Säureäquivalente je mol Hydroxylgruppe des Alkanols oder Dialkanols einsetzt,
(ii) in einer Reaktionszone zu in (i) erhaltenem wässrigen Gemisch kontinuierlich eine wässrige Lösung des anorganischen Nitrits gibt, und
(iii) gegebenenfalls die organische Phase isoliert.

2. Verfahren nach Anspruch 1, **dadurch gekennzeichnet, dass** die wässrige Mineralsäure in Schritt (i) Salzsäure ist.

3. Verfahren nach Anspruch 2, **dadurch gekennzeichnet, dass** die Konzentration von Chlorwasserstoff in Wasser 10 bis 40 Gew.-% beträgt.

4. Verfahren nach einem der vorhergehenden Ansprüche, **dadurch gekennzeichnet, dass** das anorganische Nitrit in Schritt (ii) ausgewählt ist unter Natrium- und Kaliumnitrit.

5. Verfahren nach einem der vorhergehenden Ansprüche, **dadurch gekennzeichnet, dass** man den Schritt (ii) bei Temperaturen oberhalb 5 °C bis 40 °C durchführt.

6. Verfahren nach einem der vorhergehenden Ansprüche, **dadurch gekennzeichnet, dass** das molare Verhältnis von Hydroxygruppe des Alkanols bzw. Dialkanols zu anorganischem Nitrit im Bereich von 1:1,0 bis 1:1,2 liegt.

7. Verfahren nach einem der vorhergehenden Ansprüche, **dadurch gekennzeichnet, dass** die Verweilzeit der Reaktanden in der Reaktionszone 20 Minuten bis 5 Stunden beträgt.

8. Verfahren nach einem der vorhergehenden Ansprüche, **dadurch gekennzeichnet, dass** am Reaktionsaustrag von Schritt (ii) der pH-Wert des Reaktionsgemisches oberhalb 3 liegt.

9. Verfahren nach einem der vorhergehenden Ansprüche, **dadurch gekennzeichnet, dass** das Alkanol bzw. Dialkanol ein lineares oder verzweigtes C₂-C₆-Alkanol bzw. -Dialkanol ist.

10. Verfahren nach Anspruch 9, **dadurch gekennzeichnet, dass** das C₂-C₆-Alkanol ausgewählt ist unter n-Butanol, sec.-Butanol, Isobutanol, tert.-Butanol und Isoamylalkohol.

11. Verfahren nach Anspruch 9, **dadurch gekennzeichnet, dass** das C₂-C₆-Dialkanol Neopentylglykol ist.

## Claims

1. A process for continuous preparation of alkyl nitrites and alkyl dinitrites by reaction of an alkanol or a dialkanol with an inorganic nitrite in the presence of at least one mineral acid that does not oxidize nitrite, wherein
(i) the alkanol or dialkanol is mixed with an aqueous solution of the mineral acid using on average not more than 1.01 mol of acid equivalents per mole of hydroxyl group in the alkanol or dialkanol,
(ii) an aqueous solution of the inorganic nitrite is added continuously to the aqueous mixture obtained in (i) in a reaction zone, and
(iii)the organic phase is optionally isolated.

2. The process according to claim 1, wherein the aqueous mineral acid in step (i) is hydrochloric acid.

3. The process according to claim 2, wherein the concentration of hydrogen chloride in water is in the range from 10 to 40% by weight.

4. The process according to any of the preceding claims, wherein the inorganic nitrite in step (ii) is selected from the group consisting of sodium nitrite and potassium nitrite.

5. The process according to any of the preceding claims, wherein step (ii) is carried out at temperatures above 5°C to 40°C.

6. The process according to any of the preceding claims, wherein the molar ratio of hydroxyl group in the alkanol or dialkanol to inorganic nitrite is in the range from 1:1.0 to 1:1.2.

7. The process according to any of the preceding claims, wherein the residence time of the reactants in the reaction zone is in the range from 20 minutes to 5 hours.

8. The process according to any of the preceding claims, wherein the pH of the reaction mixture at the downstream end of step (ii) is above 3.

9. The process according to any of the preceding claims, wherein the alkanol or dialkanol is a linear or branched C₂-C₆-alkanol or -dialkanol.

10. The process according to claim 9, wherein the C₂-C₆-alkanol is selected from the group consisting of n-butanol, sec-butanol, isobutanol, tert-butanol and isoamyl alcohol.

11. The process according to claim 9, wherein the C₂-C₆-dialkanol is neopentyl glycol.

## Revendications

1. Procédé pour la production en continu de nitrites d'alkyle et de dinitrites d'alkyle en faisant réagir un alcanol ou un dialcanol avec un nitrite inorganique en présence d'au moins un acide minéral n'ayant aucun effet oxydant sur le nitrite, **caractérisé en ce que** l'on :
(i) mélange de l'alcanol ou du dialcanol avec une solution aqueuse de l'acide minéral, avec en moyenne pas plus de 1,01 mole d'équivalents acide par mole de groupe hydroxyle de l'alcanol ou du dialcanol,
(ii) ajoute en continu une solution aqueuse du nitrite inorganique dans une zone réactionnelle au mélange aqueux obtenu lors de l'étape (i), et
(iii) isole éventuellement la phase organique.

2. Procédé selon la revendication 1, **caractérisé en ce que** l'acide minéral aqueux est de l'acide chlorhydrique lors de l'étape (i).

3. Procédé selon la revendication 2, **caractérisé en ce que** la concentration de chlorure d'hydrogène dans l'eau se situe entre 10 à 40 % en poids.

4. Procédé selon une des revendications précédentes, **caractérisé en ce que** le nitrite inorganique est choisi parmi le nitrite de sodium ou de potassium lors de l'étape (ii).

5. Procédé selon une des revendications précédentes, **caractérisé en ce que** l'étape (ii) est effectuée à des températures situées au-dessus de 5 °C à 40 °C.

6. Procédé selon une des revendications précédentes, **caractérisé en ce que** le ratio molaire du groupe hydroxy de l'alcanol, respectivement du dialcanol, par rapport au nitrite inorganique se situe entre 1:1,0 et 1:1,2.

7. Procédé selon une des revendications précédentes, **caractérisé en ce que** le temps de séjour des réactifs dans la zone réactionnelle est de 20 minutes à 5 heures.

8. Procédé selon une des revendications précédentes, **caractérisé en ce que** la valeur de pH du mélange réactionnel du produit de réaction de l'étape (ii) est supérieure à 3.

9. Procédé selon une des revendications précédentes, **caractérisé en ce que** l'alcanol, respectivement le dialcanol, est un alcanol respectivement un dialcanol linéaire ou ramifié en C₂-C₆.

10. Procédé selon la revendication 9, **caractérisé en ce que** l'alcanol en C₂-C₆ est choisi parmi le n-butanol, le sec- butanol, l'isobutanol, le tert-butanol et l'alcool isoamylique.

11. Procédé selon la revendication 9, **caractérisé en ce que** le dialcanol en C₂-C₆ est du néopentylglycol.
